# EUROPEAN PATENT APPLICATION

(11) **EP 1 726 301 A1**
(43) Date of publication of application: **29.11.2006**
(21) Application number: 05300407.3
(22) Date of filing: 24.05.2005
(51) Int. Cl.: A61K 9/50, A61K 45/06

(54) **Oral pharmaceutical composition for treating a COX-2 mediated condition**

(71) Applicant: FLAMEL TECHNOLOGIES, 69693 Venissieux Cédex (FR)
(72) Inventor: Soula, Gérard, 69330 Meyzieu (FR); Guimberteau, Florence, 33450 Montusson (FR)
(74) Representative: Fleurance, Raphaël

(57) **Abstract**

The invention relates to oral pharmaceutical formulations for the prevention and/or the treatment of chronic cyclooxygenase (COX)-2 mediated diseases or conditions, ie the inflammatory diseases or conditions, while reducing the risk of thrombotic cardiovascular and. This invention also addresses methods of prevention and/or treatment of these diseases, using these oral pharmaceutical formulations.

The oral pharmaceutical formulation comprises a combination of at least one active principle for treating a chronic cyclooxygenase(COX)-2 mediated disease or condition and of microcapsules for the controlled release of acetylsalicylic acid (ASA), in the gastrointestinal environment.

## Description

The invention relates to oral pharmaceutical formulations for the prevention and/or the treatment of chronic cyclooxygenase (COX)-2 mediated diseases or conditions, ie the inflammatory diseases or conditions, while reducing the risk of thrombotic cardiovascular and. This invention also addresses methods of prevention and/or treatment of these diseases, using these oral pharmaceutical formulations.

COX-2 mediated diseases of particular concern for the invention include inflammatory diseases.
Chronic cyclooxygenase (COX)-2 mediated diseases such as rheumatoid arthritis and systemic lupus erythematosis are often treated with Non Steroidal Anti Inflammatory Drugs (NSAIDs). A large number of patients, who are treated with common NSAIDs, or with more specific NSAIDs such as COX-2 inhibitors, have severe side effects. These severe side effects may include life threatening ulcers and thrombotic cardiovascular events that limit the therapeutic potential of said NSAIDs.
In addition, there is evidence that patients with chronic inflammatory conditions, such as rheumatoid arthritis and systemic lupus erythematosis, are at increased risk for thrombotic cardiovascular events.
Furthermore, many patients treated with NSAIDs or suffering from chronic COX-2 mediated disease or condition are elderly and thus are at increased risk for thrombotic cardiovascular events. Thus, it is desirable to treat such patients with appropriate antiplatelet therapy, such as low dose aspirin.

Thus, there is a need that the patients treated with NSAIDs receive antiplatelet aggregation drugs therapy, without gastric side effects.
In sum, there is a need for a therapeutic solution that combines NSAIDs, particularly COX-2 inhibitor anti-inflammatory drugs with anti-platelet aggregation drugs , without inducing gastric side effects.

In the present exposure, the "thrombotic cardiovascular events" include stroke, myocardial ischemia, myocardial infarction, angina pectoris, transient ischemic attack (TIA; amaurisis fugax), reversible ischemic neurologic deficits, and any similar thrombotic event in any vascular bed (splanchnic, renal, aortic, peripherical, etc.)

Aspirin, or AcetylSalicylic Acid (ASA), acts to prevent platelet aggregation by irreversibly inhibiting cyclooxygenase (COX-1 & COX-2, collectively known as COX). COX converts arachidonic acid to thromboxane, a potent vasoconstrictor and a platelet aggregation stimulator. Aspirin inhibits COX by acetylating it. The inhibition of COX activity by aspirin is generally irreversible.

When aspirin is absorbed from the digestive tract, it is collected by the portal vein. The portal vein then goes to the liver where the aspirin is deacetylated. Once deacetylated, aspirin no longer has the ability to acetylate COX. However, the mechanism in the liver can rapidly reach saturation causing the aspirin overflow to enter the systemic blood circulation. In the systemic circulation, aspirin that has not been deacetylated by the liver can further inhibit COX in other tissues and cells. For instance, outside the portal system, ie notably in the endothelial cells that line the vasculature and in the gastric endothelium, aspirin-induced COX-1 inhibition results in a decrease of prostacyclin, which, contrary to thromboxane, is a potent vasodilator, a platelet aggregation inhibitor and a cytoprotector. Therefore, aspirin that enters the systemic blood circulation results in inhibition of the prostacyclin and other prostaglandins, which runs counter to the desired effect on platelet aggregation, and which induces gastric side effects. This phenomenon of blind inhibition of the different prostaglandins in the organism is commonly referred to as the dilemma of aspirin. It is well known to scientists and has been widely described in the literature.

Aspirin is a very useful medication for the prevention of cardiovascular thrombotic events in patients with or those at risk for cardiovascular disease. However, there are serious side effects of aspirin administration. For example, the most common side effect is a propensity to induce gastric or intestinal ulceration, which may result in hemorrhaging. This effect occurs when acetylated aspirin inhibits COX-1 in the systemic circulation. Now, COX-1 in the systemic circulation catalyzes the biosynthesis of gastric prostaglandins that ordinarily serve as cytoprotective mucous in the intestines. So, the gastric mucosa is no longer protected by these gastric prostaglandins and it is atta.cked by gastric acid, which induces tissue damage and bleeding... The gastrointestinal effects of aspirin may be caused by its lack of selectivity between antiplatelet COX-1 inhibition and endothelial COX-1 inhibition leading to gastric mucosal effects.

In patients with established cardiovascular disease, aspirin use has been documented to decrease the risk of a primary myocardial infarction, stroke and vascular death. Secondary prevention refers to the use of aspirin to prevent cardiovascular events in patients with established cardiovascular disease such as a myocardial infarction, stroke, or angina. The use of aspirin in these individuals is recommended based on a documented decrease in future cardiovascular events and mortality. The risk for gastrointestinal injury is observed in patients being treated with aspirin, even at dosages as low as 81 mg/day for cardioprotection.

In patients with established cardiovascular disease, aspirin may have further side effects. For example, aspirin may decrease renal blood flow and the rate of glomerular filtration in patients with congestive heart failure. Therefore, acute renal failure may be precipitated. Aspirin may also promote the retention of salt and water by reducing the prostaglandin induced inhibition of both the reabsorption of chloride and the action of anti-diuretic hormone. This may cause edema in some patients who are treated with aspirin and may reduce the effectiveness of anti-hypertensive regimens. Aspirin and other COX-2 inhibitors may also increase the risk of heart disease. Metabolism of arachidonic acid by COX-2 results in the production of prostaglandins, which promotes inflammation. Therefore inhibition of COX-2 results in decreased inflammation. However, COX-2 inhibition also promotes arachidonic acid to be converted to pro-inflammatory agents such as leukotriene B4 and thromboxane A2. The resulting increase in these pro-inflammatory agents may lead to increased atherosclerosis and platelet aggregation, and other complications such as stroke and heart attack.

So, it appears that aspirin that is not metabolized by the liver may induce serious side effects.

Since the NSAIDs, and especially the COX-2 specific inhibitors, possibly combined with aspirin, may be taken by the patient for a substantial portion of his life, it is important to improve the safety profile of the treatment for the tens of millions of patients concerned today who regularly take these drugs.

Thus, it appears that there is a need in a therapeutic solution for treating the inflammatory disorders and the associated pathologies linked to platelet aggregation, , without causing the serious side effects to the patients.

In this respect, it has been previously proposed to associate low dose aspirin, with COX-2 inhibitors to make an anti-inflammatory therapeutic for decreasing the risk of a thrombotic cardiovascular event while decreasing the side effects (notably gastric) of such treatment.

For instance, WO-A-03/094924 suggests replacing low dose aspirin with nitric oxide releasing aspirin. WO-A-03/094924 discloses a method for treating a chronic cyclooxygenase-2 mediated disease or condition and reducing the risk of a thrombotic cardiovascular event in a human patient in need of such treatment and at risk of a thrombotic cardiovascular event comprising orally concomitantly or sequentially administering to said patient a cyclooxygenase-2 selective inhibitor in an amount effective to treat the cyclooxygenase-2 mediated disease or condition and nitric oxide releasing aspirin in an amount effective to reduce the risk of the thrombotic cardiovascular event while maintaining a high level of upper gastrointestinal safety and tolerability. The invention also encompasses a method for treating a chronic cyclooxygenase-2 mediated disease or condition and reducing the risk of a thrombotic cardiovascular event in a human patient in need of such treatment and at risk of a thrombotic cardiovascular event. This treatment comprises orally concomitantly or sequentially administering to the patient a nitric oxide releasing cyclooxygenase-2 selective inhibitor in an amount effective to treat the cyclooxygenase-2 mediated disease or condition and aspirin in an amount effective to reduce the risk of the thrombotic cardiovascular event, while maintaining a high level of upper gastrointestinal safety and tolerability. WO-A-03/033001 further proposes to use aspirin in lower dosages as those previously implemented, namely 75-325 mg per day. The particularly preferred COX-2 inhibitor is 5-alkyl-2-aminophenylacetic acid derivative.

However the immediate release aspirin included in this composition may still create many of the gastric side effects mentioned before. The problem of aspirin dilemma is not solved.

US-B-6,599,529 relates to an oral pharmaceutical modified-release multiple-units composition for the administration of a therapeutically and/or prophylactically effective amount of a non-steroid anti-inflammatory drug substance - e.g. aspirin - (in the following abbreviated "an NSAID substance") to obtain both a relatively fast or quick onset of the therapeutic effect and the maintenance of a therapeutically active plasma concentration for a relatively long period of time. The modified release multiple-units composition comprises at least two fractions of multiple units such as a first and a second fraction. The first fraction is an immediate release immediate release form of NSAIDs, which comprises individual units that are designed to quickly release the drug substance. The second fraction is a delayed release DR / sustained release SR form, which comprises individual units that are designed to slowly release the drug substance to enable a delayed and extended release of the drug substance. Typically, the second fraction comprises multiple units which are coated with a sustained release coating designed to release the drug substance in such a manner that the maintenance of a therapeutically active plasma concentration for a relatively long period of time are obtained (once- or twice-a-day administration)- [Pellet cores: Polysorbate 20/Cellulose microcrystalline/Lactose/Carmellose sodium/Maltodextrin/Pregelatinized starch - Inner coat: Hypromellose(Methocel E prem) / Magnesium stearate / Talc / Eudragit NE 30 D - Outer coat: Hypromellose (Methocel E5 prem) / Talc].The composition can comprise a further active drug substance selected from the group consisting of an antidepressant, an opioid, a prostaglandin analog, a glucocorticosteroid, a cytostaticum, a H2 receptor antagonist, a proton pump inhibitor and an antacid.

The immediate release fraction of NSAIDs (aspirin) in this composition is still creating all gastric side effects mentioned before. The problem of aspirin dilemma is not solved.

US-A-2004/0121004 and US-A-20040131676 disclose a non-enterically coated dosage form comprising: a) a proton pump inhibitor(lansoprazole); b) a buffer; and c) a non-steroidal anti-inflammatory drug (aspirin at an amount between 50 mg and 100 mg) and a method of treating a condition selected from the group consisting of angina, aorto-pulmonary shunt occlusion, colorectal cancer, esophageal cancer, colon cancer, coronary artery disease, dementia, dysmenorrhea, myocardial infarction, rheumatoid arthritis, osteoarthritis, pain, headache, migraine headache, stroke, thrombocythemia, post-operative thromboembolism, ischemia, bursitis, cognitive decline, fever, gout, musculoskeletal disorders, soft tissue injury, and pericarditis; wherein the method comprises administering to a patient having one or more of the above conditions said non-enterically coated dosage form. This form is an immediate release form of acetylsalicylic acid at basic pH. Granulates of NSAIDs are prepared from Magnesium Hydroxide- buffer-; Calcium Carbonate; Mannitol; Avicel (micro-crystalline Cellulose); PVPP (Cross- povidone). These granulates are tabletted.

The immediate release of NSAIDs (aspirin) in this composition is still creating all gastric side effects mentioned before. The problem of aspirin dilemma is not solved.

US-B-5,603,957 belonging to the applicant, and incorporated in its entirety by reference, discloses a pharmaceutical form comprising microcapsules for the controlled release of acetylsalicylic acid in the gastrointestinal environment, said microcapsules consisting of particles of acetylsalicylic acid with a size of between 100 and 1000 µm. These microcapsules are coated and designed so that, when ingested orally in a single administration of a dose of between 50 and 325 mg of ASA, they induce moderate acetylsalicylic acid absorption kinetics in vivo in man, extending over at least 24 hours, said acetylsalicylic acid absorption being less than or equal to 10% by weight of the absorbed fraction of D at a time t after ingestion of 0.4 hour, less than or equal to 50% by weight of the absorbed fraction of D at t=3.9 hours, and less than or equal to 90% by weight of the absorbed fraction of D at t=23 hours, t being given to within +/-10%. One method to create such smaller microcapsules is disclosed in U.S. Patent No. 6,022,562 to Autant et al., which is incorporated in its entirety by reference.

We find it surprising that we can use the pharmaceutical of microcapsules to selectively inhibit the cyclooxygenase COX-1 in the portal vein and/or in the liver, and thus reduce the production of thromboxane, while minimizing cyclooxygenase COX-1 inhibition in the systemic circulation, in order to to optimize the inhibition of platelet aggregation, and subsequently to prevent and/or treat cardiovascular diseases and risks associated with anti-inflammatory drugs used in the treatment of chronic cyclooxygenase (COX)-2 mediate diseases or conditions and consisting of NSAIDs, more particularly of COX-2 specific inhibitors, while minimizing the side effects (notably the gastric ones).

While not wishing to be constrained by any mode of action, we believe that by coating the acetylsalicylic acid to form microcapsules, we promote the direct action of aspirin on only the COX-1 of the portal circulation. Our pharmaceutical formulation results in a low, constant release rate of aspirin being released from the microcapsules and absorbed by the portal vein. This low release rate of aspirin is sufficient to inhibit COX-1 in the portal circulation, and therefore prevent thromboxane formation. Once aspirin is deacetylated, it is no longer active in that it can no longer inhibit COX. The liver can quickly be saturated with aspirin, and any aspirin that is not deacetylated will overflow into the systemic blood stream. We find it surprising that our use of microcapsules results in a release rate of aspirin low enough such that the liver is not saturated. Therefore, once the low release rate of aspirin inhibits COX-1 in the portal circulation, the liver then deacetylates any remaining active aspirin. This results in minimal aspirin overflow into the systemic circulation to inhibit prostaglandin and prostacyclin production, thus preventing damage of the gastroendothelium and other side effects of aspirin. Thus, the method permits the rapid saturation of the liver without the normal deleterious side effects, a result that we fmd unexpected.

Optionally, we also propose to add a minimal dose of a gastric acid suppressing agent to the above mentioned combination of NSAIDs with a controlled release form of aspirin that releases aspirin at a low release rate such that very little aspirin reaches the systemic circulation. This gastric acid suppressing agent is intended to decrease or suppress any residual gastric damage by increasing gastric pH

While not wishing to be constrained to low doses of gastric acid suppressing agent, we fmd it surprising that we can greatly minimize gastric damage with only a minimal dose of gastric acid suppressing agent. The low dose of gastric acid suppressing agent in our invention increases the gastric pH only a small amount. Further, the low, constant dose of aspirin released in the intestinal tract would sufficiently inhibit COX in the portal circulation, while having minimal effects on the systemic prostaglandins. Therefore, the applicant takes credit for demonstrating that the combination of NSAIDs with a controlled release aspirin microcapsules and with at least one gastric acid suppressing agent makes it possible to increase the safety of the anti-platelet drug while decreasing the side effects.

According to the invention, we propose the combination of:
- at least one NSAIDs, preferably a COX-2 specific inhibitor,
- a controlled release dosage form of aspirin releasing slowly acetylsalicylic acid in such manner that a few amount of acetylsalicylic acid reaches the systemic circulation and, hence, induces very limited gastric damage, and,
- and optionally minimal amounts of gastric acid suppressing agent to completely suppress the gastric damage without important increase of the gastric pH.

The present invention concerns an oral pharmaceutical formulation for treating a chronic cyclooxygenase(COX)-2 mediated disease or condition while reducing the risk of a thrombotic cardiovascular event in a human patient in need of such treatment and at risk of a thrombotic cardiovascular event,
- comprising a combination of at least one active principle for treating a chronic cyclooxygenase(COX)-2 mediated disease or condition and of microcapsules for the controlled release of acetylsalicylic acid (ASA), in the gastrointestinal environment,
- said microcapsules:
   - having an in vitro release profile, in 0.05M potassium dihydrogenophosphate/sodium hydroxide buffer medium pH 6.8, such that 70% of the acetylsalicylic acid is released over a period of time of between 2 and 20 hours, , and
   - inhibiting cyclooxygenase COX-1 in portal vein which limits the production of thromboxane while maintaining cyclooxygenase COX-1 in systemic blood stream and hence limiting the inhibition of the production of prostaglandin and prostacyclin, to protect the gastric endothelium and to maintain its vasodilatation properties.

The term "controlled release" denotes, in the present disclosure, a prolonged or sustained release and/or a delayed release and/or a pulsed release of active principle by an oral pharmaceutical formulation. Such a controlled-release oral pharmaceutical formulation may, for example, comprise an immediate-release phase and a slow-release phase. Modified-release medicinal products are well known in this field; see, for example, Remington: The Science and practice of pharmacy", 19th edition, Mack Publishing Co. Pennsylvania, USA. The modified release may in particular be a prolonged and/or delayed release.

The oral pharmaceutical formulation according to the invention can be, e.g., a once-α-day or a twice-α-day form.

Preferably, the active principle for treating a chronic cyclooxygenase-2 mediated disease or condition in the oral pharmaceutical formulation according to the invention is an anti-inflammatory drug. More preferably, the active principle for treating a chronic cyclooxygenase-2 mediated disease or condition in the oral pharmaceutical formulation according to the invention is selected from the Non Steroidal Anti Inflammatory Drugs (NSAIDs).

For instance, the NSAID(s) is (are) selected in the group comprising:
- aminoarylcarboxylic acid and its derivates such as: enfenamic acid, flufenamic acid, isonixin, meclofenamic acid, mefenamic acid, morniflumate, niflumic acid and tolfenamic acid,
- arylacetic acid and its derivates such as: aceclofenac, acemetacin, amfenac, bromfenac, cimmetacin, diclofenac, etodolac, fentiazac, glucametacin, indomethacin, lonazolac, l'acid metiavinic, oxametacine, pirazolaque, proglumetacin, sulindac, tiaramide, tolmetin and zomepirac,
- arylcarboxylic acids such as ketorolac and tinoridine,
- arylpropionic acid and its derivates such as: alminoprofen, bermoprofen, carprofen, dexibuprofen, fenbufen, fenoprofen, flunoxaprofen, flurbiprofen, ibuprofen, ibuproxam, ketoprofen, loxoprofen, naproxen, oxaprozin, pranoprofen, protizinic acid and tiaprofenic acid,
- pyrazoles e.g.: epirizole,
- pyrazolones such as: benzpiperylon, mofebutazone, oxyphenbutazone, phenylbutazone and ramifenazone,
- salicylic acid derivates comme : acetaminosalol, benorylate, eterisalate, fendosal, imidazole salicylate, lysine acetylsalicylate, morpholine salicylate, parsalmide, l'acid salamidacetic and le salsalate,
- thiazinecarboxamides such as : ampiroxicam, droxicam, lornoxicam, meloxicam, piroxicam and tenoxicam,
- others such as: bucillamine, bucolome, bumadizon, diferenpiramide, ditazol, emorfazone, nabumetone, nimesulide, proquazone and piroxicam (in the form of the betacyclodextrin complex).
- alclofenac, azapropazone, benoxaprofen, acid bucloxic, choline magnesium trisalicylate, clidanaque, clopinaque, dapsone, diflunisal, fenclofenec, floctafenine, flufenisal, (r)-flurbiprofen, (s)-flurbiprofen, furofenaque, feprazone, fluprofen, ibufenaque, indoprofen, isoxepac, isoxicam, miroprofen, mefenamic, meclofen, acid niflumic, nitroflurbiprofen, oxipinaque, podophyllotoxin derivates, piprofen, pirprofen, prapoprofen, sudoxicam, suprofen, acid tiaprofenic, tiopinaque, tioxaprofen, zidometacin, acid 2-fluoro-a-methyl[1,1'-biphenyl]-4-acetic, a 4-(nitrooxy)butyl ester, ketoporfen, ketrolac.

More particularly: the NSAID(s) is (are) selected in the group comprising:
lornoxicam, diclofenac, nimesulide, ibuprofen, piroxicam, piroxicam (betacyclodextrin), naproxen, ketoprofen, tenoxicam, aceclofenac, indometacin, nabumetone, acemetacin, morniflumate, meloxicam, flurbiprofen, acid tiaprofenic, proglumetacin, acid mefenamic, fenbufen, etodolaque, tolfenamic acid, sulindac, phenylbutazone, fenoprofen, tolmetin, acetylsalicylic acid, dexibuprofen and/or the pharmaceutical salts and/or the complexes and/or the prodrugs and/or the mixtures thereof.

More preferably, in the oral pharmaceutical formulation according to the invention, the active principle for treating a chronic cyclooxygenase-2 mediated disease or condition, is selected in the sub-class of the class of NSAIDs comprising the specific inhibitors of COX-2.

The examples of cyclooxygenase-2 specific or selective inhibitors include: rofecoxib (VIOXX® cf. US-B- 5 474 995), etoricoxib (ARCOXIA™ cf. US-B- 5 861 419), celecoxib (CELEBREX® cf. US-B- 5 466 823), valdecoxib (cf. US -B-6 633 272), parecoxib (cf. brevet US -B-5 932 598), COX-189 (Novartis), BMS347070 (Bristol Myers Squibb), tiracoxib ou JTE522 (Japan Tobacco), ABT963 (Abott), CS502 (Sankyo), and GW406381 (GlaxoSmithKline).

With respect to therapeutic agents, it is expected that the skilled practitioner will adjust dosages on a case by case basis using methods well established in clinical medicine. The daily dosage may be provided in either a single or multiple regimen with the latter being generally preferred. These are simply guidelines since the actual dose must be carefully selected and titrated by the attending physician based upon clinical factors unique to each patient. The optimal daily dose will be determined by methods known in the art and will be influenced by factors such as the age of the patient, the disease state, side effects associated with the particular agent being administered and other clinically relevant factors.

Generally, the COX-2 inhibitor may be present between about 1 to about 1000mg , preferably about 5 to about 500mg. For example, the recommended dosage for one particular COX-2 inhibitor, Celecoxib (Celebrex), is typically 100 mg twice per day or 200 mg once per day. Celecoxib is a preferred COX-2 inhibitor in the compositions and methods of the present invention and may typically be present at 50-500 mg per unit dose. Especially preferred are methods and compositions utilizing 100 to 400 mg celecoxib. As another example, Rofecoxib (Vioxx) for oral administration is available in tablets of 12. 5, 25 or 50 mg and in an oral suspension containing either 12.5 mg or 25 mg rofecoxib per 5 ml. The recommended initial daily dosage for the management of acute pain is 50 mg. Peak plasma concentrations of rofecoxib typically occur about 2-3 hours after oral administration and the drug has a half life of about 17 hours.

Advantageously, the oral pharmaceutical COX-2 inhibitor formulations, can include:
→ at least one immediate COX-2 inhibitor form;
→ and/or at least one controlled release COX-2 inhibitor form.

According to an aspect of the invention, the oral pharmaceutical formulation comprises at least one gastric acid suppressing agent to increase the pH of the stomach just enough to minimize the damage resulting from the residual amount of non-deacetylated acetylsalicylic acid coming from the liver and the portal blood circulation and entering the systemic blood circulation. Preferably, said gastric acid suppressing agent is a proton pump inhibitor (ppi).

Preferably, the microcapsules can be orally ingestible in a dose and can comprise particles of acetylsalicylic acid with a size of less than about 1000 µm, preferably between about 50 µm or 100 µm to 1000 µm, which are coated and designed so that, when ingested orally in a single administration they induce acetylsalicylic acid absorption kinetics in vivo in man, extending over at least 24 hours, said acetylsalicylic acid absorption being less than or equal to 10% by weight of the absorbed fraction of the dose at about 0.4 hour post ingestion, less than or equal to 50% by weight of the absorbed fraction of the dose at about 3.9 hours post ingestion, and less than or equal to 90% by weight of the absorbed fraction of the dose at about 23 hours post ingestion.

According to another of its features, the present invention also concerns a method for treating a chronic cyclooxygenase(COX)-2 mediated disease or condition and reducing the risk of a thrombotic cardiovascular event in a human patient in need of such treatment and at risk of a thrombotic cardiovascular event, said method comprising the administration to a patient an oral pharmaceutical formulation according to the above definition. This method is a method of preventing and/or treating pathological disorders associated with excesses of thromboxane, particularly cardiovascular diseases and risks. This method consists in the oral administration of the pharmaceutical formulation according to the invention, preferably in a once or twice-a-day administration.

It is apparent from the foregoing text that the microcapsules of the invention are very effective in pharmacological terms, perfectly tolerated by the organism, especially as regards gastric tolerance, capable of being presented in various appropriate galenical forms and, finally, easy and inexpensive to obtain.

The controlled release acetylsalicylic acid microcapsules have high selectivity for the thromboxane inhibition, which makes it possible to maintain the production of prostacyclin, in order to protect the gastro-intestinal tract.

Furthermore, the gastric acid suppressing agent maintains the pH of the stomach high enough to reduce the acidic erosion of the surface of the stomach and even to facilitate the healing process when some ulceration occurs.

In a preferred embodiment of the invention, the absorption takes place over a period of between 24 and hours in the following manner:
- 10% of the absorbed fraction of the dose at t=0.4 to 5 hours,
- 50% of the absorbed fraction of the dose at t=3.9 to 25 hours,
- and 90% of the absorbed fraction of the dose at t=23 to 45 hours.

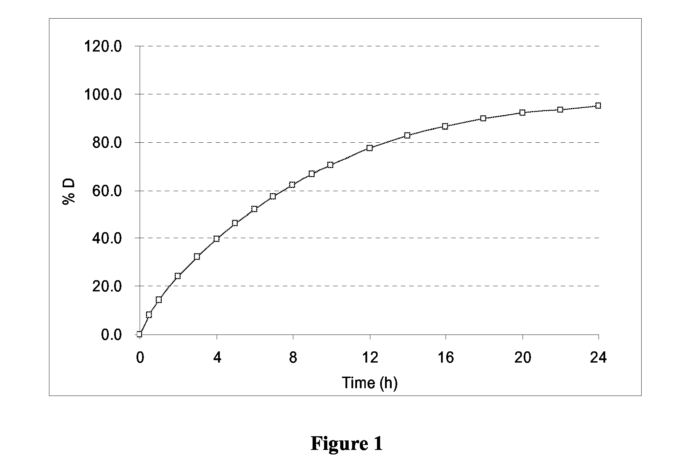

The curve of FIG. 1 of US-B-5,603,957 shows the kinetic profile of the in vivo absorption of ASA, and more precisely the upper limit of the acetylsalicylic acid in vivo absorption profile induced by the controlled release acetylsalicylic acid microcapsules according to US-B-5,603,957, as a function of time, at a dose of 320 mg. This absorption is expressed in % absorbed relative to the absorbed fraction of the initial dose D. This curve is obtained by conventional deconvolution analysis (Milo GIBALDI and D. PERRIER, Pharmacokinetics, 2nd ed., New York, Marcel Dekker Inc., 1983, p. 145-167) from the mean curves of the plasma concentrations as a function of time after the oral administration of 350 mg of acetylsalicylic acid equivalents of ASPEGIC. (control form) and 320 mg of acetylsalicylic acid equivalents of microcapsules according to the invention in the form of gelatin capsules. In this case, the tracer molecule chosen for the plasma concentrations as a function of time is necessarily salicylic acid (SA), a metabolite of ASA. The plasma concentrations of SA are determined by HPLC. The critical points at 0.4, 3.9 and 23 h, given above in the defmition of the microcapsules of the invention, are of course to be found on this curve. Beyond this curve, the hepatic acetylsalicylic acid deacetylation mechanism is saturated. It must be considered that all the acetylsalicylic acid in vivo absorption profiles contained in the area under the curve are controlled release acetylsalicylic acid microcapsules according to US-B-5,603,957.

To solve the technical problem on which the invention is based, it highly preferable that the formulation according to the invention be free or almost free of immediate release acetylsalicylic acid form. The term "almost free" means that the formulation can include negligible amount of immediate release acetylsalicylic acid form, namely a insufficient amount so that there is remaining non-deacetylated acetylsalicylic acid in the systemic blood stream after the liver, said non-deacetylated acetylsalicylic acid being in a sufficient amount to inhibit COX-1 of the systemic blood compartment.

"Immediate release acetylsalicylic acid form" is intended to denote, in the present disclosure, a form in which the acetylsalicylic acid is released, at pH 6.8 and under sink conditions in an in vitro dissolution test, most of the amount of acetylsalicylic acid contained in the immediate release acetylsalicylic acid form, in a relatively brief period of time; for example at least 70% of the acetylsalicylic acid is preferably released in forty five minutes and more preferably in thirty minutes.

All the dissolution profiles to which reference is made in the present disclosure are determined according to the indications of the European Pharmacopoeia, 4th edition, entitled: "Dissolution test for solid oral forms": type II dissolutest performed under SINK conditions, at 37°C, at a test dose of 10 mg of active, and with agitation of 100 rpm.

In a preferred embodiment of the invention, the oral pharmaceutical formulation according to the invention contains a dose of acetylsalicylic acid in the controlled release acetylsalicylic acid microcapsules, said dose being comprised between 60 and 320 mg.

In another preferred embodiment of the invention, the oral pharmaceutical formulation according to the invention contains a dose of acetylsalicylic acid in the controlled release acetylsalicylic acid microcapsules, said dose being comprised between 50 and 325 mg.

In a more preferred embodiment of the invention, the oral pharmaceutical formulation according to the invention contains a dose of acetylsalicylic acid in the controlled release acetylsalicylic acid microcapsules, said dose being comprised between 75 and 310 mg.

In a preferred embodiment of the invention, the oral pharmaceutical formulation according to the invention, contains a dose of gastric acid suppressing agent comprised between 1 and 130 mg.

In a more preferred modality of the invention, the oral pharmaceutical formulation according to the invention, contains a dose of gastric acid suppressing agent comprised between 2 and 120 mg.

According to a first embodiment, the oral pharmaceutical formulation according to the invention has the following characteristics, among others:
a) the dose of NSAID(s) is comprised between 1 and 1000 mg;
b) the dose of acetylsalicylic acid in the controlled release acetylsalicylic acid microcapsules is comprised between 50 and 325 mg;
c) optionally, the dose of gastric acid suppressing agent is comprised between 5 and 120 mg;
d) it is a once-a-day administration form.

The oral pharmaceutical formulation comprising a), b), optional c), d) features, can include:
■ at least one immediate release NSAID(s) form
■ and/or at least one controled release NSAID(s) form
■ and/or at least one immediate release gastric acid suppressing agent form
■ and/or at least one controlled release gastric acid suppressing agent form.

According to a second embodiment, the oral pharmaceutical formulation according the invention has the following characteristics, among others:
i. the dose of NSAID(s) is comprised between 1 and 1000 mg;
ii. wherein the dose of acetylsalicylic acid in the microcapsules is comprised between 50 and 325 mg;
iii. wherein the optional dose of gastric acid suppressing agent is comprised between 5 and 120 mg;
iv. which is a twice-a-day administration form.

The oral pharmaceutical formulation comprising i, ii, optional iii, iv features, can include:
■ at least one immediate release NSAID(s) form
■ and/or at least one controled release NSAID(s) form
■ and/or at least one immediate release gastric acid suppressing agent form
■ and/or at least one controlled release gastric acid suppressing agent form.

It is remarkable that the oral pharmaceutical formulation according to the invention, is designed so that it induces reduction of bleeding and pitichia, or ulceration, in the stomach during the treatment.

We find it surprising that we can selectively inhibit COX-1 in the portal vein, permitting minimal aspirin to enter the systemic circulation. This significantly increases the comfort of the patients and the safety of the drug. Patients will be no longer compelled either to interrupt the treatment with aspirin or to switch to another drug. This also permits a method of quickly inhibiting COX in the portal vein, with less side effects than previously known. Finally, we have a method that permits the unexpected precise titration of the effects of aspirin on the liver, the circulatory system, and the stomach.

This remarkable feature can give rise to a method for reducing the side effects during the treatment of diseases at least partially caused by an inhibition of COX-1 in systemic circulation, said treatment comprising the administration to a patient of an oral pharmaceutical formulation for treating chronic cyclooxygenase(COX)-2 mediated diseases or conditions and reducing the risk of a thrombotic cardiovascular event in a human patient in need of such treatment and at risk of a thrombotic cardiovascular event, said formulation comprising a combination of at least one active principle for treating a chronic cyclooxygenase(COX)-2 mediated disease or condition and of microcapsules for the controlled release of acetylsalicylic acid (ASA), in the gastrointestinal environment,
said microcapsules:
• having an in vitro release profile, in 0.05M potassium dihydrogenophosphate/sodium hydroxide buffer medium pH 6.8, such that 70% of the acetylsalicylic acid is released over a period of time of between 2 and 20 hours, and
• inhibiting cyclooxygenase COX-1 in portal vein which limits the production of thromboxane while maintaining cyclooxygenase COX-1 in systemic blood stream and hence limiting the inhibition of the production of prostaglandin and prostacyclin, to protect the gastric endothelium and to maintain its vasodilatation properties.

According to a preferred embodiment, said formulation comprises at least one gastric acid suppressing agent increasing the pH of the stomach just enough to minimize the damage resulting from the residual amount of non-deacetylated acetylsalicylic acid coming from the liver and the portal blood circulation and entering the systemic blood circulation.

The side effects of the oral pharmaceutical formulation according to the invention is also very interesting in that it is designed so that it improves the healing process, notably, in the stomach during the treatment. The oral pharmaceutical formulation is also designed to decrease other side effects of antithrombotic treatments, such as gastric or intestinal ulceration and hemorrhaging, renal failure, edema, atherosclerosis, and any resulting cardiovascular disease.

This interesting feature can give rise to a method according to another aspect of the present invention, namely a method for improving the healing process, notably, in the stomach, during the treatment of diseases at least partially caused by an inhibition of COX-1 in systemic circulation, said treatment comprising the administration to a patient of an oral pharmaceutical formulation including ASA, wherein the oral pharmaceutical formulation is a formulation for treating chronic cyclooxygenase(COX)-2 mediated diseases or condition and reducing the risk of a thrombotic cardiovascular event in a human patient in need of such treatment and at risk of a thrombotic cardiovascular event,
said formulation comprising a combination of at least one active principle for treating a chronic cyclooxygenase(COX)-2 mediated disease or condition and of microcapsules for the controlled release of acetylsalicylic acid (ASA), in the gastrointestinal environment, said microcapsules:
• having an in vitro release profile, in 0.05M potassium dihydrogenophosphate/sodium hydroxide buffer medium pH 6.8, such that 70% of the acetylsalicylic acid is released over a period of time of between 2 and 20 hours, , and
• inhibiting cyclooxygenase COX-1 in portal vein which limits the production of thromboxane while maintaining cyclooxygenase COX-1 in systemic blood stream and hence limiting the inhibition of the production of prostaglandin and prostacyclin, to protect the gastric endothelium and to maintain its vasodilatation properties.

According to a variant which makes it possible to decrease the possible residual gastric damage resulting from the passage of possible remaining small amounts of non deacetylated ASA from the portal and/or the liver into the systemic circulation, said formulation comprises at least one gastric acid suppressing agent increasing the pH of the stomach just enough to minimize the damage resulting from the residual amount of non-deacetylated acetylsalicylic acid coming from the liver and the portal blood circulation and entering the systemic blood circulation.

In the two above mentioned methods, the implemented microcapsules being orally ingestible in a dose and comprising particles of acetylsalicylic acid with a size of less than 1000 µm which are coated and designed so that, when ingested orally in a single administration of a dose of acetylsalicylic acid, it induces acetylsalicylic acid absorption kinetics in vivo in man, extending over at least 24 hours, said acetylsalicylic acid absorption being less than or equal to 10% by weight of the absorbed fraction of the dose at about 0.4 hour post ingestion, less than or equal to 50% by weight of the absorbed fraction of the dose at about 3.9 hours post ingestion, and less than or equal to 90% by weight of the absorbed fraction of the dose at about 23 hours post ingestion.

Unless otherwise indicated, use of the term "about" in this invention description is intended to mean plus or minus 10% of the designated amount; thus, "about 5 to 80%" would mean a range of 4.5-5.5% to 76-84%.

The gastric acid suppressing agent is preferably a proton pump inhibitor (ppi). According to the terminology of the present text, the acronym "ppi" used in the singular will designate indifferently one or several ppi, e.g. the lansoprazole, and /or at least one of its metabolites.

A ppi is a substituted benzimidazole which inhibits gastric acid secretions by specific inhibition of the H⁺, K⁺-ATPase enzymatic system (proton pump) of the secretory surface of parietal gastric cells. A ppi is an advantageous substitute for histamine H2 receptor antagonists (blocking of gastric acid secretion) or for antacids, which are not fully effective in the treatment of ulcers, associated or not with *Helicobacter pylori* infection or of other gastric disorders, and which in addition lead to many side effects.

A ppi is a lipophilic weak base that is poorly soluble in water. It undergoes rapid degradation under acidic conditions but, on the other hand, it is relatively stable at neutral or basic pH.

The ppi more particularly concerned by the present invention are derivates of benzimidazole. These latter are substituted or non substituted benzimidazoles, one or several salts of benzimidazoles, any enantiomer of these benzimidazoles, one or several salts of enantiomer (s), any isomer of these benzimidazoles, any derivate of benzimidazole, any free base of benzimidazole ou any mixing of these active principles.

The ppi used in the dosage forms of the invention may be used in neutral form or in the form of an alkaline salt, such as for instance the Mg++, Ca++, Na++, K++ or Li++ salts, preferably the Mg++ salts. Further where applicable, the compounds listed above may be used in racemic form or in the form of a substantially pure enantiomer thereof, or alkaline salts of the single enantiomers.

For example, the ppi concerned by the invention, are notably the ppi described pages 7 to 11 of WO-A-97/25066, this extract being incorporated by reference in the present text.

The WO-A-2004/035020 patent application gives also a general formula of the class of benziimidazoles: pages 35-48. This extract of WO-A-2004/035020 is incorporated by reference in the present text.

Examples of ppi are: esomeprazole, leminoprazole, omeprazole, pantoprazole, pariprazole, rabeprazole, timoprazole, picoprazole and tenatoprazole.

Suitable proton pump inhibitors are for example disclosed in EP-A1-0005129, EP-A1-174 726, EP-A1-166 287, GB 2 163 747 and WO90/06925, WO91/19711, WO91/19712, and further especially suitable compounds are described in WO95/01977 and W0094/27988.

The gastric acid suppressing agent is preferably a proton pump inhibitor, but H₂ receptor antagonists such as ranitidine, cimetidine or famotidine may be used in the pharmaceutical compositions with an alginate as proposed in WO 95/017080 or together with antacid agent(s). A wide variety of antacid agent(s) and/or alginates may be used in combination with a suitable proton pump inhibitor in the fixed unit dosage form according to the present invention. Such antacid agents include for example aluminium hydroxide, calcium carbonate, magnesium hydroxide, magnesium carbonate and aluminium magnesium hydroxide carbonate (hydrotalcit) taken alone or in combinations with each other. The alginates may be an alginate selected from alginic acid or sodium alginate or other pharmaceutically acceptable alginate salts, hydrates, esters etc. Especially preferred antacid agents are magnesium or calcium based antacid agents and aluminium hydroxide/magnesium carbonate complex. Suitable antacid agents are for instance described in U.S. Pat. No. 5,409,709.

The preferred ppi in the form of a racemate, an alkaline salt or one of its single enantiomers, optionally in combination with antacid agent(s), can be an immediate release form and/or a controlled release (CR) form.

As examples of CR/MR, reference can be made to individually enteric or non enteric coating layered individual units (small beads, granules, microcapsules or pellets).

For example, the gastric acid suppressing agent can be designed in the form of CR/MR microcapsules, notably of the type of the CR/MR acetylsalicylic acid microcapsules, as described herein.

It could be very useful that the oral pharmaceutical formulation according to the invention, comprises at least one active principle different from acetylsalicylic acid and from gastric acid suppressing agent - preferably a proton pump inhibitor (ppi).

According to one embodiment, the oral pharmaceutical formulation further comprises a cardiovascular selected from the group comprising: anti-platelet drugs, beta adrenergic receptor blockers, calcium channel blockers, angiotensin converting enzyme inhibitors, diuretics, anti-arrhythmic drugs, anti-ischemic drugs, anti-hypertensive drugs, beta adrenergic agonists, cardiac glycosides, nitrates, sodium channel blockers, central nervous system acting anti-hypertensive drugs, potassium channel activators, vasodilatory drugs, vasoconstrictive drugs, and mixtures thereof.

Examples of anti-platelet drugs include non-steroidal anti-inflammatory drugs, dipyridamole, and ticlopidine.

Examples of diuretics include acetazolamide, dichlorphenamide, methazolamide, glycerin, isosorbide, mannitol, urea, furosemide, bumetanide, ethacrynic acid, torsemide, azosemide, muzolimine, piretanide, tripamide, bendroflumethiazide (naturetin), benzthiazide (exna), chlorothiazide (diuril), hydrochlorothiazide (hydrodiuril), hydroflumethiazide (saluron), methyclothiazide (enduron), polythiazide (renese), trichlormethiazide, chlorthalidone (hygroton), indapamide (lozol), metolazone (mykrox, zaroxolyn), quinethazone (hydromox), amiloride, triamterene, spironolactone, canrenone, potassium canrenoate.

Examples of angiotensin converting enzyme inhibitors include benazepril, captopril, enalapril, fosinopril sodium, lisinopril, quinapril, ramipril, spirapril.

Examples of nitrates include amyl nitrite (isoamyl nitrite), nitroglycerin (glyceryl trinitrate, nitro-bid, nitrostat, nitrol, nitro-dur, others), isosorbide dinitrate (isordil, sorbitrate, dilatrate, others), isosorbide-5-mononitrate (imdur, ismo, others), erythrityl tetranitrate (cardilate).

Examples of calcium channel blockers include amlodipine (norvasc), bepridil (vascor), diltiazem (cardizem, dilacor), felodipine (plendil), isradipine (dynacirc), nicardipine (cardene), nifedipine (adalat, procardia), nimodipine (nimotop), verapamil (calan, isoptin, verelan).

Examples of vasodilator drugs include nitrovasodilators such as nitroglycerin, isosorbide dinitrate, sodium nitroprusside; angiotensin receptor antagonist such as losartan (cozaar), phosphodiesterase inhibitors such as amrinone (inocor), milrinone (primacor), and vesnarinone; "direct" vasodilators such as hydralazine (apresoline), nicorandil, adrenergic receptor antagonists such as prazosin (minipress, and other quinazoline derivatives), phentolamine (regitine), labetalol (normodyne, trandate), carvedilol, and bucindolol; Ca²⁺ channel blocking drugs such as nifedipine (adalat, procardia), amlodipine (norvasc), and sympathomimetics such as dobutamine (dobutrex).

Examples of anti-arrhythmic drugs include adenosine, amiodarone, bretylium, digoxin, digitoxin, diltiazem, disopyramide, esmolol, flecainide, lidocaine, mexiletine, moricizine, phenytoin, procainamide (N-acetyl procainamide), propafenone, propranolol, quinidine, sotalol, tocainide, verapamil.

According to another embodiment, the oral pharmaceutical formulation comprises an anti-diabetic drug. Examples of anti-diabetic drugs include: Acarbose, Acetohexamide, Buformin, 1-Butyl-3-metanilylurea, Carbutamide, Chlorpropamide, Ciglitazone, Glibornuride, Gliclazide, Glimepiride, Glipizide, Gliquidone, Glisoxepid, Glyburide, Glybuthiazole, Glybuzole, Glyhexamide, Glymidine, Glypinamide, Metformin, Miglitol, Nateglinide, Phenbutamide, Phenformin, Pioglitazone, Proinsulin, Repaglinide, Rosiglitazone, Tolazamide, Tolbutamde, Tolcyclamide, Troglitazone and/or the pharmaceutical salts and/or the complexes and/or the prodrugs and/or the mixtures thereof.

Advantageously, the controlled release acetylsalicylic acid microcapsules are so designed that the absorption takes place over a period of between 24 and 48 hours in the following manner: 10% of the absorbed fraction of the dose at t=0.4 to 5 hours, 50% of the absorbed fraction of the dose at t=3.9 to 25 hours, and 90% of the absorbed fraction of the dose at t=23 to 45 hours.

In the disclosure of the invention, the term "controlled release acetylsalicylic acid microcapsules" denotes microparticles of acetylsalicylic acid that are film-coated with at least one coating for modified/controlled release of ASA. The non-film-coated microparticles of acetylsalicylic acid may, for example, be neutral cores coated with at least one layer containing ASA, or microparticles of pure acetylsalicylic acid or alternatively granules formed by a matrix of support excipients including lansoprazole.

Advantageously, the film-coating (or coating) covering(s) has(have) sufficient mechanical strength to prevent it(them) tearing and/or it(them) breaking up in the organism, until the end of the release of the active principle.

These controlled release acetylsalicylic acid microcapsules can be compared to vehicles for the transport and the release of acetylsalicylic acid and, optionally, of one or more other active principles in the stomach and in the small intestine.

The pharmaceutical formulation according to the invention can also be characterized in that the controlled release acetylsalicylic acid microcapsules have an in vitro release profile, in 0.05M potassium dihydrogenophosphate/sodium hydroxide buffer medium pH 6.8, such that:
• 70% of the acetylsalicylic acid is released over a period of time of between 1 and 10 hours, preferably between 2 and 8 hours, and even more preferably between 2 and 6 hours, and
• 40% of the acetylsalicylic acid is released over a period of time of between 0.5 and 5 hours, preferably between 1 and 4 hours, and even more preferably between 1 and 3 hours.

Advantageously, the controlled release acetylsalicylic acid microcapsules have an in vitro release profile, in a 0.04M hydrochloric acid medium pH 1.4, such that 40% of the acetylsalicylic acid is released over a period of time of less than or equal to 3h, preferably less than or equal to 2h, and even more preferably less than or equal to 0.75h.

According to another pharmacokinetic definition of the pharmaceutical formulation according to the invention, the controlled release acetylsalicylic acid microcapsules have an in vitro release profile in 0.05M potassium dihydrogenophosphate/sodium hydroxide buffer medium pH 6.8, such that, for any value of time t of between 2h and t(70%), preferably for any value of time t of between 1h and t(70%), the % of dissolved (released) acetylsalicylic acid is greater than or equal to 35 t / t(70%).

For example, the coating of the controlled release acetylsalicylic acid microcapsules represents 5 to 50% by weight, based on the total mass of said microcapsules.

Preferably, the coating of the controlled release acetylsalicylic acid microcapsules comprises at least one layer which controls the modified release of agent, the composition of said layer is as follows:
A) at least one film-forming (co)polymer (A) that is insoluble in the fluids of the gastrointestinal tract;
B) optionally, at least one water-insoluble hydrophilic film-forming (co)polymer (B) that is insoluble in the fluids of the gastrointestinal tract, carrying groups that are ionized in the fluids of the gastrointestinal tract,
C) at least one (co)polymer (C) that is soluble in the fluids of the gastrointestinal tract;
D) at least one plasticizer (D);
E) optionally, at least one surfactant and/or lubricant (E).

According to a preferred embodiment of the invention:
* (A) is selected from the group of following products:
   - non-water-soluble derivatives of cellulose, preferably ethylcellulose and/or cellulose acetate,
   - polyvinyl acetates,
   - and mixtures thereof.
* (B) is chosen from water-insoluble charged acrylic derivatives, preferably from (co)polymers of acrylic and methacrylic acid ester carrying at least one quaternary ammonium group, (B) even more preferably comprising at least one copolymer of alkyl (meth)acrylate and of trimethylammonioethyl methacrylate chloride, and more precisely the products sold under the trade marks EUDRAGIT ® RS and/or RL, e.g. the powders EUDRAGIT ® RL PO and/or EUDRAGIT ® RS PO and/or the granules EUDRAGIT ® RL 100 and/or EUDRAGIT ® RS 100 and/or the suspensions and/or solutions of these EUDRAGIT ® RL and RS, namely, respectively, EUDRAGIT® RL 30D and/or EUDRAGIT® RS 30D and/or EUDRAGIT® RL 12.5 and/or EUDRAGIT® RS 12.5;
* (C) is chosen from
   - nitrogenous (co)polymers, preferably from the group comprising polyacrylamides, poly-N-vinylamides, polyvinylpyrrolidones (PVP) and poly-N-vinyllactams;
   - water-soluble derivatives of cellulose,
   - polyvinyl alcohols (PVAs),
   - polyoxyethylenes (POEs),
   - and mixtures thereof;
   polyvinylpyrrolidone being particularly preferred.
* (D) is chosen from the group comprising:
   - cetyl alcohol esters,
   - glycerol and its esters, preferably from the following subgroup: acetylated glycerides, glyceryl monostearate, glyceryl triacetate, glyceryl tributyrate,
   - phthalates, preferably from the following subgroup: dibutyl phthalate, diethyl phthalate, dimethyl phthalate, dioctyl phthalate,
   - citrates, preferably from the following subgroup: acetyl tributyl citrate, acetyltriethyl citrate, tributyl citrate, triethyl citrate,
   - sebacates, preferably from the following subgroup: diethyl sebacate, dibutyl sebacate,
   - adipates,
   - azelates,
   - benzoates,
   - plant oils,
   - fumarates, preferably diethyl fumarate,
   - malates, preferably diethyl malate,
   - oxalates, preferably diethyl oxalate,
   - succinates, preferably dibutyl succinate,
   - butyrates,
   - salicylic acid,
   - triacetin,
   - malonates, preferably diethyl malonate,
   - castor oil (this being particularly preferred),
   - and mixtures thereof.
* (E) is chosen from the group comprising:
   - anionic surfactants, preferably from the subgroup of alkali metal or alkaline-earth metal salts of fatty acids, stearic acid and/or oleic acid being preferred,
   - and/or nonionic surfactants, preferably from the following subgroup:
      ■ polyoxyethylenated oils, preferably polyoxyethylenated hydrogenated castor oil,
      ■ polyoxyethylene-polyoxypropylene copolymers,
      ■ polyoxyethylenated esters of sorbitan,
      ■ polyoxyethylenated derivatives of castor oil,
      ■ stearates, preferably calcium stearate, magnesium stearate, aluminium stearate or zinc stearate,
      ■ stearyl fumarates, preferably sodium stearyl fumarate,
      ■ glyceryl behenates,
      ■ and mixtures thereof.

According to a particularly advantageous embodiment, the composition of the modified-release layer is as follows:
A. the film-forming polymer(s) (A) is (are) present in a proportion of 10 to 90%, preferably 20 to 40% by weight on a dry basis relative to the total mass of the coating composition;
B. the water-insoluble hydrophilic film-forming polymer(s) (B) is (are) present in a proportion of 10 to 90%, preferably 20 to 40% by weight on a dry basis relative to the total mass of the coating composition;
C. the polymer(s) (C) that is (are) soluble in the fluids of the gastrointestinal tract is (are) present in a proportion of 2 to 25, preferably 5 to 15% by weight on a dry basis relative to the total mass of the coating composition;
D. the plasticizer(s) (D) is (are) present in a proportion of 2 to 20, preferably of 4 to 15% by weight on a dry basis relative to the total mass of the coating composition;
E. the optional surfactant(s) and/or lubricant(s) (E) is (are) present in a proportion of 2 to 20, preferably of 4 to 15% by weight on a dry basis relative to the total mass of the coating composition.

For further details, in particular qualitative and quantitative details, regarding at least some of the constituents of this coating composition, reference will be made, for example, to European patent EP-B-0 709 087 or to PCT applications WO-A-2004/010983 and WO-A-2004/010984, the content of which are incorporated into the present disclosure, in their entirety, by reference.

The monolayer or multilayer coating may comprise various other additional adjuvants conventionally used in the coating field. They may be, for example, pigments or coloring agents, fillers, or anti-foaming agents.

To prevent the problems of caking of the coated particles constituting the controlled release acetylsalicylic acid microcapsules used in the invention, provision is advantageously made for adding thereto at least one anticaking agent preferably formed of talc, colloidal silica or a mixture of the two.

According to a particular embodiment of the invention, the controlled release acetylsalicylic acid microcapsule coating responsible for the modified release of acetylsalicylic acid consists of a single coating layer or a single coating film. This simplifies their preparation and limits the degree of coating.

Moreover, the pharmaceutical formulation according to the invention has the particularity that the coating of each controlled release acetylsalicylic acid microcapsule is nonenteric and does not desintegrate whatever the pH be, and in particular at any pH above 5.0.

Advantageously, the diameter of the controlled release acetylsalicylic acid microcapsules is less than or equal to 1000 µm, preferably between 50 and 800 µm, and even more preferably between 100 and 600 µm. The microparticle diameters to which the present disclosure refers are, unless otherwise indicated, mean diameters by volume.

This size makes it possible to cross the stomach independently of the opening of the pylorus. The gastric transit time is thus shorter and more uniform.

Advantageously, the controlled release acetylsalicylic acid microcapsules are obtained from particles of acetylsalicylic acid having a size of between 250 and 800 µm before the coating operation.

According to the invention, practical implementations in which the proportion of acetylsalicylic acid in the microcapsules (expressed as % by weight on a dry basis relative to the total mass of the microcapsules) is between 5 and 80, preferably between 10 and 60, and even more preferably between 20 and 50, are preferred.

As regards the monolayer or multilayer coating (or film) responsible for the modified release of ASA, it represents, for example, at most 40%, preferably at most 15%, by weight of the microcapsules.

The controlled release acetylsalicylic acid microcapsules are obtained from particles of acetylsalicylic acid which are coated by being sprayed with the intimate combination forming the coating, suspended in an organic solvent or mixture of organic solvents. The coating process, which constitutes a further subject of the invention, fits into the general pattern of microencapsulation techniques, of which the main ones are summarized in the article by C. DUVERNEY and J. P. BENOIT in "L'actualite chimique", December 1966. More precisely, the technique in question is microencapsulation by film coating. Preferably, this process consists essentially in: a) preparing the coating composition by mixing ABCDE in a solvent system, b) applying the composition/solvent system mixture to particles of acetylsalicylic acid, c) drying the resulting microcapsules, and d) if appropriate, mixing the latter with at least one anticaking agent. Examples of solvents which are suitable for forming part of the composition of the solvent system are ketones, esters, chlorinated solvents, alcohols, preferably aliphatic alcohols, alkanes or mixtures thereof. These solvents are advantageously C₁-C₆ compounds and particularly preferably acetone, methyl ethyl ketone, methanol, ethanol, isopropanol, cyclohexane and methylene chloride. If the coating methodology which can be used according to the invention is considered in greater detail, it can be stated that the coating composition/solvent system mixture is applied by being sprayed onto the moving particles of ASA, said movement preferably being created by mechanical agitation or by blowing (fluidization). To obtain microcapsules according to the invention possessing the desired absorption kinetics, it is necessary to encapsulate particles of acetylsalicylic acid with a mean size of between 75 and 500 µm, preferably of between 300 and 500 µm, for a dose of between 75 and 320 mg.

The controlled release acetylsalicylic acid microcapsules described above, which may have been obtained by the process also explained above, can be used for the preparation of novel galenical forms of aspirin having a biochemical selectivity for the inhibition of thromboxane relative to the other prostaglandins, in particular for the preparation of novel galenical forms useful as platelet aggregation inhibitors, and/or, more precisely, for the preparation of novel galenical forms active in the prevention and/or treatment of cardiovascular diseases and risks.

The present invention further relates to these novel galenical units as such, and comprising the pharmaceutical formulation according to the invention.

Advantageously, these galenical, novel in their structure, their presentation and their composition, are presented in the form of a sachet of powder, a sachet of a powder for multidose suspension to be reconstituted, a tablet or a gelatin capsule. They can contain, for instance, a dose of acetylsalicylic acid of 20 to 500 mg, preferably 50 to 400 mg and particularly preferably 50 to 325 mg of acetylsalicylic acid and a dose of ppi of 1 to 300 mg, preferably 2 to 200 mg and particularly preferably 5 to 120 mg. Such galenical forms are preferably administered in single or twice daily doses.

It should be noted that it can be of value to mix, in one and the same gelatin capsule, tablet or powder, at least two types of microcapsules whose absorption kinetics are different but within the framework characteristic of the controlled release acetylsalicylic acid microcapsules according to US-B-5,603,957 (profile of curve of FIG. 1).

The invention will be understood more clearly from the following Examples, which are given solely by way of illustration and serve to provide a clear understanding of the invention and to illustrate its different embodiments and/or modes of implementation, as well as its various advantages.

### EXAMPLES

### Description of the figure:

Figure 1: In vitro release profiles for the CR-Aspirin-based Microcapsules prepared according to Example 1.

### Example 1 Preparation of CR-Aspirin-based microcapsules

66 g of ethyl cellulose (Ethocel 7 Premium / Dow), 7 g of Plasdone K29/32® (Povidone/ISP), 8 g of castor oil, 9 g of magnesium stearate and 10 g tartaric acid are dispersed in 1200 g of a mixture made of 60% of isopropanol & 40% of acetone. The suspension is sprayed on 900 g of acetylsalicylic acid (aspirin) crystals, previously sieved between 200 and 500 µm.
These microcapsules have been tested in a pH 6.8 (KH₂PO₄ 0.05M/ NaOH) dissolution medium maintained at 37°C and stirred with a paddle speed of 100 rpm (USP II apparatus) (See Figure 1).

### Example 2: Preparation of IR- celecoxib based microcapsules

860 g of celecoxib, 70 g of Klucel EF® (Hydroxypropyl cellulose / Aqualon) & 70 of Lutrol F-68 (Poloxamer 188 / BASF) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.
These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Example 3: Preparation of IR-rofecoxib based microcapsules

1500 g of rofecoxib, 150 g de Klucel EF® (Hydroxypropyl cellulose / Aqualon) and 150 g of Cremophor RH 40® (PEG 40-hydrogenated castor oil / BASF) are dispersed in 4000 g of water. The suspension is sprayed on 200 g of neutral microspheres (Asahi-Kasei) in a spray coater Glatt GPCG1.
These microcapsules have been tested in a pH 6.8 (KH₂PO₄ 0.05M/ NaOH) dissolution medium maintained at 37°C and stirred with a paddle speed of 100 rpm (USP II apparatus). Their release is immediate.

### Example 4: Preparation of IR-meloxicam based microcapsules

600 g of meloxicam, 100 g de Klucel EF® (Hydroxypropyl cellulose / Aqualon) and 100 g of Lutrol F-68 (Poloxamer 188 / BASF) are dispersed in 2000 g of water. The suspension is sprayed on 200 g of neutral microspheres (Asahi-Kasei) in a spray coater Glatt GPCG1.
These microcapsules have been tested in a pH 6.8 (KH₂PO₄ 0.05M/ NaOH) dissolution medium maintained at 37°C and stirred with a paddle speed of 100 rpm (USP II apparatus). Their release is immediate.

### Example 5: Preparation of enteric coated- lansoprazole based microcapsules

### Step 1:

900 g of lansoprazole & 100 g of Klucel EF® (Hydroxypropyl cellulose / Aqualon) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.

### Step 2:

50 g of Eudragit L100-55® (Rohm) and 10 g of triethyl citrate are dispersed in isopropanol. This solution is sprayed on 450 g of lansoprazole granules (prepared at step 1).
These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Example 6: Capsule containing CR-aspirin and IR- celecoxib

180 mg of microcapsules of acetylsalicylic acid prepared in example 1 (i.e. 162.5 mg of acetylsalicylic acid) and 233 mg of microcapsules of celecoxib (i.e. 200 mg of celecoxib) prepared at example 2 are filled in size 0 capsule.
This capsule is the fmal dosage form for preventing cardiovascular diseases (by means of aspirin), said diseases being caused by repeated administration of anti-inflammatory drugs, such as COX-2 inhibitors (celecoxib).

### Example 7: Capsule containing CR-aspirin and IR- rofecoxib

180 mg of microcapsules of acetylsalicylic acid prepared in example 1 (i.e. 162.5 mg of acetylsalicylic acid) and 33 mg of microcapsules of rofecoxib (i.e. 25 mg of rofecoxib) prepared at example 3 are filled in size 2 capsule.
This capsule is the fmal dosage form for preventing cardiovascular diseases (by means of aspirin), said diseases being caused by repeated administration of anti-inflammatory drugs, such as COX-2 inhibitors (rofecoxib).

### Example 8: Capsule containing CR-aspirin and IR-meloxicam

180 mg of microcapsules of acetylsalicylic acid prepared in example 1 (i.e. 162.5 mg of acetylsalicylic acid) and 25 mg of microcapsules of meloxicam (i.e. 15 mg of meloxicam) prepared at example 4 are filled in size 2 capsule.
This capsule is the fmal dosage form for preventing cardiovascular diseases (by means of aspirin), said diseases being caused by repeated administration of anti-inflammatory drugs, such as COX-2 inhibitors (meloxicam).

### Example 9: Capsule containing CR-aspirin, enteric coated-lansoprazole and IR-celecoxib

180 mg of microcapsules of acetylsalicylic acid prepared in example 1 (i.e. 162.5 mg of acetylsalicylic acid), 6.2 mg of microcapsules of lansoprazole (i.e. 5 mg of lansoprazole) prepared at example 5 and 233 mg of microcapsules of celecoxib (i.e. 200 mg of celecoxib) prepared at example 2 are filled in size 0 capsule.
This capsule is the final dosage form for preventing cardiovascular diseases (by means of aspirin), said diseases being caused by repeated administration of anti-inflammatory drugs, such as COX-2 inhibitors (celecoxib), while hindering gastric damages due to the presence of a ppi and controlled release acetylsalicylic acid microcapsules. The combination of a ppi with the controlled release acetylsalicylic acid microcapsules makes it possible to prevent gastric damages due to aspirin.

### Example 10 showing that the known controlled release acetylsalicylic acid microcapsules could be improved, by means of the combination according to the invention:

Controlled release microcapsules of aspirin according to example 1 were compared to aspirin at a dose of 325 mg in double blind, randomized cross-over study, on 24 healthy non smoking volunteers. Endoscopic damage was assessed on days 0, 7, 14, 21 on each treatment period. The primary end point was the total number of gastroduodenal erosions and petechiae assessed endoscopically. The study performed by a group led by Professor Hawkey of Gastroenterology Division of University Hospital, Nottingham (UK) indicated that Controlled released aspirin cause less endoscopic damage than conventional aspirin.

The study indicated that significantly fewer gastric lesions were observed in patients taking controlled released aspirin 325 mg than in patients taking entero-coated aspirin at the same dose. In particular, gastric erosion per patient were 1.57 with Controlled released aspirin 325 compared to 5.48 with entero-coated aspirin product, or a reduction of 70% (p<0.001). Concerning haemorrhagic event, 0.3 events per patient were observed with controlled released aspirin 325 compared to 2.96 with conventional aspirin (p<0.001). In addition, a 3.09 petechia per patient were observed with controlled released aspirin compared to 7.35 with the comparator (p<0.001).
These very positive results for controlled released aspirin could be explained by the biochemical selectivity of the product which inhibits platelets COX-1 and consequently thromboxan (TXB2), the platelet aggregant prostanoids, which sparing prostacyclin (PGI2), the systemic cytoproctective prostaglandin generated by endothelial COX-1.

Although, this result is very positive for GI tract safety, it open the door to an improvement of the controlled released aspirin directed toward a decrease of the level of safety events especially those observed in gastro intestinal tract. It is worthwhile noticing that if the number of adverse events is significantly decreased using Controlled released aspirin compared to conventional aspirin, the GI tact events (i.e. erosion and petechia) are still measurable and could led to some safety concerns for chronic use.

The embodiments illustrated and discussed in this specification are intended only to teach those skilled in the art the best way known to the inventors to make and use the invention. Nothing in this specification should be considered as limiting the scope of the present invention. Modifications and variations of the above-described embodiments of the invention are possible without departing from the invention, as appreciated by those skilled in the art in light of the above teachings. It is therefore to be understood that, within the scope of the claims and their equivalents, the invention may be practiced otherwise than as specifically described, which merely illustrate preferred embodiments.

## Claims

1. An oral pharmaceutical formulation notably for treating a chronic cyclooxygenase(COX)-2 mediated disease or condition and reducing the risk of a thrombotic cardiovascular event in a human patient in need of such treatment and at risk of a thrombotic cardiovascular event,
• comprising a combination of at least one active principle for treating a chronic cyclooxygenase(COX)-2 mediated disease or condition and of microcapsules for the controlled release of acetylsalicylic acid (ASA), in the gastrointestinal environment,
• said microcapsules:
- having an in vitro release profile, in 0.05M potassium dihydrogenophosphate/sodium hydroxide buffer medium pH 6.8, such that 70% of the acetylsalicylic acid is released over a period of time of between 2 and 20 hours, and
- inhibiting cyclooxygenase COX-1 in portal vein which limits the production of thromboxane while maintaining cyclooxygenase COX-1 in systemic blood stream and hence limiting the inhibition of the production of prostaglandin and prostacyclin, to protect the gastric endothelium and to maintain its vasodilatation properties.

2. An oral pharmaceutical formulation according to claim 1, wherein the active principle for treating a chronic cyclooxygenase-2 mediated disease or condition, is selected in the class of the Non Steroidal Anti Inflammatory Drugs (NSAIDs).

3. An oral pharmaceutical formulation according to claim 1 or 2, wherein the active principle for treating a chronic cyclooxygenase-2 mediated disease or condition, is selected in the sub-class of the class of NSAIDs comprising the inhibitors of COX-2.

4. An oral pharmaceutical formulation according to claim 1, comprising at least one gastric acid suppressing agent increasing the pH of the stomach just enough to minimize the damage resulting from the residual amount of non-deacetylated acetylsalicylic acid coming from the liver and the portal blood circulation and entering the systemic blood circulation.

5. An oral pharmaceutical formulation according to claim 4, wherein the gastric acid suppressing agent is a proton pump inhibitor (ppi).

6. An oral pharmaceutical formulation according to claim 1, wherein the microcapsules being orally ingestible in a dose and comprising particles of acetylsalicylic acid with a mean diameter below or equal to 1000 µm, which are coated and designed so that, when ingested orally in a single administration of a dose of between 50 and 325 mg of ASA, they induce moderate acetylsalicylic acid absorption kinetics in vivo in man, extending over at least 24 hours, said acetylsalicylic acid absorption being less than or equal to 10% by weight of the absorbed fraction of D at a time t after ingestion of 0.4 hour, less than or equal to 50% by weight of the absorbed fraction of D at t=3.9 hours, and less than or equal to 90% by weight of the absorbed fraction of D at t=23 hours, t being given to within +/-10%.

7. An oral pharmaceutical formulation according to claim 1, wherein the dose of acetylsalicylic acid in the microcapsules is comprised between 60 and 320 mg, preferably between 75 and 310 mg.

8. An oral pharmaceutical formulation according to claim 4, wherein the dose of gastric acid suppressing agent is comprised between 1 and 130 mg, preferably between 2 and 120 mg.

9. An oral pharmaceutical formulation according to claim 1 and 4:
a) the dose of acetylsalicylic acid in the controlled release acetylsalicylic acid microcapsules is comprised between 50 and 325 mg;
b) the dose of gastric acid suppressing agent is comprised between 5 and 120 mg;
c) it is a once-a-day administration form.

10. An oral pharmaceutical formulation according to claim 9 including at least one immediate release gastric acid suppressing agent form.

11. An oral pharmaceutical formulation according to claim 1 and 4:
i. wherein the dose of acetylsalicylic acid in the microcapsules is comprised between 50 and 325 mg;
ii. wherein the dose of gastric acid suppressing agent is comprised between 5 and 120 mg;
iii. which is a twice-α-day administration form.

12. An oral pharmaceutical formulation according to claim 11 including at least one immediate release acetylsalicylic acid form.

13. An oral pharmaceutical formulation according to claim 11 including at least one immediate release gastric acid suppressing agent form.

14. An oral pharmaceutical formulation according to claim 1, which is designed so that it induces reduction of bleeding and pitichia, notably, in the stomach during the treatment.

15. An oral pharmaceutical formulation according to claim 1, which is designed so that it improves the healing process, notably, in the stomach during the treatment.

16. An oral pharmaceutical formulation according to claim 1, comprising at least one active principle different from ASA, gastric acid suppressing agent and active principle for treating a chronic cyclooxygenase(COX)-2 mediated disease or condition.

17. An oral pharmaceutical formulation according to claim 16, wherein the active principle different from ASA, gastric acid suppressing agent and active principle for treating a chronic cyclooxygenase(COX)-2 mediated disease or condition, is selected in the group comprising:
• the cardiovascular drugs acting as anti-platelets;
• the cardiovascular drugs acting as beta-blockers;
• the cardiovascular drugs acting as ACE inhibitors;
• and any of their associations.

18. An oral pharmaceutical formulation according to claim 16, wherein the active principle different from acetylsalicylic acid and gastric acid suppressing agent is selected in the group of the anti-inflammatory drugs.

19. An oral pharmaceutical formulation according to claim 16, wherein the active principle different from acetylsalicylic acid gastric acid suppressing agent and active principle for treating a chronic cyclooxygenase(COX)-2 mediated disease or condition, is selected in the group of the anti-diabetic drugs.

20. An oral pharmaceutical formulation according to claim 6, wherein the acetylsalicylic acid microcapsules are so designed that the absorption takes place over a period of between 24 and 48 hours in the following manner:
10% of the absorbed fraction of D at t=0.4 to 5 hours, 50% of the absorbed fraction of D at t=3.9 to 25 hours, and 90% of the absorbed fraction of D at t=23 to 45 hours.

21. An oral pharmaceutical formulation according to claim 1, wherein the coating agent of the acetylsalicylic acid microcapsules represents 5 to 50% by weight, based on the total mass of said microcapsules.

22. An oral pharmaceutical formulation according to claim 1, wherein the coating of the acetylsalicylic acid microcapsules comprises at least one layer which controls the modified release of agent, the composition of said layer is as follows:
A. at least one film-forming (co)polymer (A) that is insoluble in the fluids of the gastrointestinal tract;
B. optionally, at least one water-insoluble hydrophilic film-forming (co)polymer (B) that is insoluble in the fluids of the gastrointestinal tract, carrying groups that are ionized in the fluids of the gastrointestinal tract,
C. at least one (co)polymer (C) that is soluble in the fluids of the gastrointestinal tract;
D. at least one plasticizer (D);
E. optionally, at least one surfactant and/or lubricant (E).

23. An oral pharmaceutical formulation according to claim 22, wherein:
* (A) is selected from the group of following products:
- non-water-soluble derivatives of cellulose,
- polyvinyl acetates,
- mixtures thereof;
* (B), when it is present, is chosen from water-insoluble charged acrylic derivatives,;
* (C) is chosen from
- nitrogenous (co)polymers;
- water-soluble derivatives of cellulose,
- polyvinyl alcohols (PVAs),
- polyoxyethylenes (POEs),
- and mixtures thereof;
* (D) is chosen from the group comprising:
- cetyl alcohol esters,
- glycerol and its esters,
- phthalates, citrates, sebacates, adipates,
- azelates,
- benzoates,
- plant oils,
- fumarates, malates, oxalates, succinates,
- butyrates,
- salicylic acid,
- triacetin,
- malonates,
- castor oil ,
- and mixtures thereof;
* (E) is chosen from the group comprising:
- anionic surfactants,
- and/or nonionic surfactants.

24. An oral pharmaceutical formulation according to claim 33, wherein the composition of the modified-release layer is as follows:
A. the film-forming polymer(s) (A) is (are) present in a proportion of 10 to 90%, by weight on a dry basis relative to the total mass of the coating composition;
B. the hydrophilic water-insoluble film-forming polymer(s) (B) is (are) present in a proportion of 0 to 90%, by weight on a dry basis relative to the total mass of the coating composition;
C. the soluble polymer(s) (C) is (are) present in a proportion of 2 to 25, by weight on a dry basis relative to the total mass of the coating composition;
D. at least one plasticizer (D) is (are) present in a proportion of 2 to 20, by weight on a dry basis relative to the total mass of the coating composition;
E. the optional surfactant(s) and/or lubricant(s) (E) is (are) present in a proportion of 2 to 20, by weight on a dry basis relative to the total mass of the coating composition.

25. An oral pharmaceutical formulation according to claim 1, wherein the diameter of the acetylsalicylic acid microcapsules is less than or equal to 1000 µm,.

26. An oral pharmaceutical formulation according to claim 1, wherein the acetylsalicylic acid microcapsules are obtained from particles of acetylsalicylic acid having a size of between 250 and 800 µm before the coating operation.

27. An oral pharmaceutical formulation according to claim 1, wherein the proportion of acetylsalicylic acid in the microcapsules (expressed as % by weight on a dry basis relative to the total mass of the microcapsules) is between 5 and 80.

28. An oral pharmaceutical formulation according to claim 1, provided in the form a galenical unit selected in the group comprising: a sachet of powder, a sachet of a powder for multidose suspension to be reconstitued, a tablet or a gelatin capsule.
